(19) Europäisches Patentamt
European Patent Office
Office européen des brevets

(11) EP 3 943 182 A1

(12) EUROPEAN PATENT APPLICATION
published in accordance with Art. 153(4) EPC

(43) Date of publication:
**26.01.2022 Bulletin 2022/04**

(21) Application number: **20773440.1**

(22) Date of filing: **13.03.2020**

(51) International Patent Classification (IPC):
***B01D 71/40*** (2006.01) ***C08F 8/32*** (2006.01)
***C08F 8/34*** (2006.01) ***C08F 265/06*** (2006.01)
***C08F 2/44*** (2006.01) ***B01D 15/38*** (2006.01)
***C08G 75/045*** (2016.01) ***B01J 20/26*** (2006.01)
***B01J 20/281*** (2006.01) ***B01J 20/30*** (2006.01)

(52) Cooperative Patent Classification (CPC):
**B01D 15/38; B01D 71/40; B01J 20/26; B01J 20/281; B01J 20/30; C08F 2/44; C08F 8/32; C08F 8/34; C08F 265/06; C08G 75/045**

(86) International application number:
**PCT/JP2020/011306**

(87) International publication number:
**WO 2020/189593 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.03.2019 JP 2019052188**
**20.03.2019 JP 2019052189**
**29.08.2019 JP 2019156263**
**29.08.2019 JP 2019156264**

(71) Applicant: **Mitsubishi Chemical Corporation**
**Chiyoda-ku**
**Tokyo 100-8251 (JP)**

(72) Inventors:
- **UWATOKO Miku**
  **Tokyo 100-8251 (JP)**
- **YASUDA Noriyuki**
  **Tokyo 100-8251 (JP)**
- **TAKEHARA Jun**
  **Tokyo 100-8251 (JP)**
- **OHARA Shohei**
  **Tokyo 100-8251 (JP)**
- **FUKUDA Yoshito**
  **Tokyo 100-8251 (JP)**

(74) Representative: **Ter Meer Steinmeister & Partner Patentanwälte mbB**
**Nymphenburger Straße 4**
**80335 München (DE)**

(54) **POLYMER, SEPARATING AGENT, PRODUCTION METHOD OF POLYMER, SEPARATION METHOD OF COMPOUND, AND PRODUCTION METHOD OF COMPOUND**

(57) The present invention relates to a polymer including at least one structure selected from the group consisting of a structure represented by General Formula (3) described below and a structure represented by General Formula (4) described below:

EP 3 943 182 A1

[Chemical Formula 1]

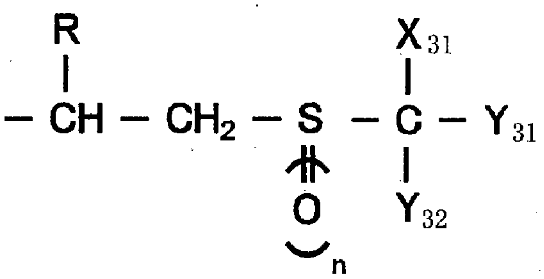

(3)

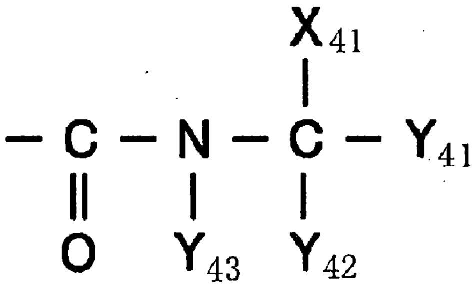

(4)

in General Formula (3) and General Formula (4) described above, $X_{31}$ and $X_{41}$ represent a hydrophilic group-containing structure, n represents an integer of 0 to 2, R represents a hydrogen atom or an alkyl group, $Y_{31}$ to $Y_{32}$ and $Y_{41}$ to $Y_{43}$ each independently represent a hydrophilic group-containing structure, a hydrogen atom, or an alkyl group.

## Description

### TECHNICAL FIELD

The present invention relates to a polymer. In addition, the invention relates to a separating agent, a production method of a polymer, a separation method of a compound, and a production method of a compound.

### BACKGROUND ART

A (meth)acrylic polymer is excellent in transparency, machine characteristics, and workability, and thus, has been widely used in various fields such as an optical material, a vehicular material, a lighting material, an architectural material, and a coating material. Among them, porous particles or a porous film of the (meth)acrylic polymer is excellent in separation capacity and machine characteristics, and thus, has been used for separating a compound.

In a case where the porous particles or the porous film of the (meth)acrylic polymer which has been used for separating the compound has high hydrophobicity on the surface, irreversible adsorptive accumulation of a target that is represented by protein easily occurs, and a recovery rate of the target decreases or fine pores are blocked.

For this reason, in order to relieve the hydrophobicity of the (meth)acrylic polymer, many hydrophilizing methods of the (meth)acrylic polymer have been considered. For example, in Patent Document 1, a method of copolymerizing a hydrophilic monomer is disclosed. In addition, in Patent Document 2 and Patent Document 3, a method of copolymerizing a monomer having a functional group and of bonding a hydrophilic compound to the functional group is disclosed.

### CITATION LIST

### PATENT DOCUMENT

Patent Document 1: JP S60-55009 A
Patent Document 2: JP S54-160300 A
Patent Document 3: JP 2014-210888 A

### SUMMARY OF THE INVENTION

### PROBLEM TO BE SOLVED BY THE INVENTION

However, in the method disclosed in Patent Document 1, it is necessary to increase a content rate of the hydrophilic monomer in order to hydrophilize the (meth)acrylic polymer to be obtained, polymerization is not easily controlled, and the purification of the (meth)acrylic polymer to be obtained is complicated. In addition, in the method disclosed in Patent Document 2 and Patent Document 3, a special monomer is necessary in order to hydrophilize the (meth)acrylic polymer to be obtained, and a complicated step is required for producing the polymer.

The invention has been made in consideration of such circumstances of the related art described above, and an object thereof is to provide a production method of a polymer that can be industrially practically used in which a (meth)acrylic polymer can be simply hydrophilized with a small number of steps.

In addition, another object of the invention is to provide a polymer and a separating agent having excellent hydrophilicity.

### MEANS FOR SOLVING PROBLEM

As a result of intensive studies of the present inventors in order to attain the objects described above, it has been found that the objects described above can be attained, and thus, the invention has been completed.

That is, the invention relates to <1> to <16> described below.

<1> A polymer including at least one structure selected from the group consisting of a structure represented by General Formula (3) described below and a structure represented by General Formula (4) described below:

[Chemical Formula 1]

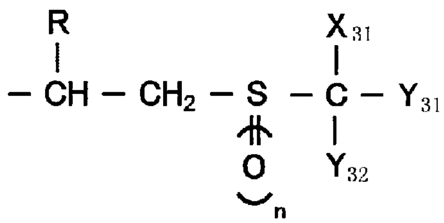

(3)

in General Formula (3) described above, $X_{31}$ represents a hydrophilic group-containing structure, $Y_{31}$ and $Y_{32}$ each independently represent a hydrophilic group-containing structure, a hydrogen atom, or an alkyl group, n represents integer of 0 to 2, and R represents a hydrogen atom or an alkyl group; and

[Chemical Formula 2]

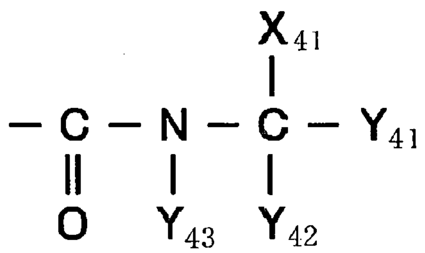

(4)

in General Formula (4) described above, $X_{41}$ represents a hydrophilic group-containing structure, and $Y_{41}$ to $Y_{43}$ each independently represent a hydrophilic group-containing structure, a hydrogen atom, or an alkyl group.

<2> The polymer according to <1>, in which the hydrophilic group includes at least one selected from the group consisting of a hydroxyl group, a carboxyl group, a sulfo group, and an amino group.

<3> A separating agent including at least one structure selected from the group consisting of a structure represented by General Formula (1) described below and a structure represented by General Formula (2) described below:

[Chemical Formula 3]

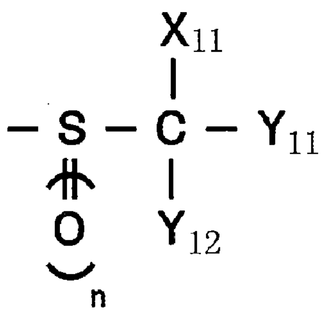

(1)

in General Formula (1) described above, $X_{11}$ represents a hydrophilic group-containing structure, $Y_{11}$ and $Y_{12}$ each independently represent a hydrophilic group-containing structure, a hydrogen atom, or an alkyl group, and n represents an integer of 0 to 2; and

[Chemical Formula 4]

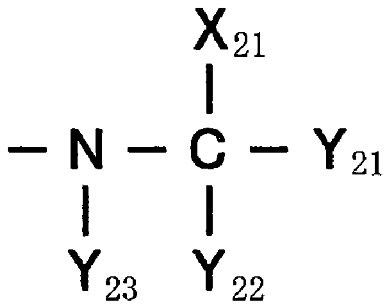

(2)

in General Formula (2) described above, $X_{21}$ represents a hydrophilic group-containing structure, and $Y_{21}$ to $Y_{23}$ each independently represent a hydrophilic group-containing structure, a hydrogen atom, or an alkyl group.

<4> The separating agent according to <3>, in which the hydrophilic group includes at least one selected from the group consisting of a hydroxyl group, a carboxyl group, a sulfo group, and an amino group.

<5> A production method of a polymer, including at least one method selected from the group consisting of methods (1) to (3) described below:

a method (1) of obtaining a polymer (C1) by reacting a (meth)acrylic polymer (A) with a thiol compound (B1) having a hydrophilic group in accordance with a thiol-ene reaction;
a method (2) of obtaining a polymer (C2) by reacting the (meth)acrylic polymer (A) with an aminoalcohol compound (B2) in accordance with an ester exchange reaction; and
a method (3) of obtaining a polymer (C3) by polymerizing a monomer (B3) including at least one selected from the group consisting of a sulfonic acid having a vinyl group and N-substituted (meth)acrylamide in the presence of the (meth)acrylic polymer (A).

<6> The production method of a polymer according to <5>, in which the polymer (A) includes a cross-linked structure.

<7> The production method of a polymer according to <5> or <6>, in which in the method (1), the polymer (C1) is obtained through an oxidation step after the reaction between the polymer (A) and the compound (B1).

<8> The production method of a polymer according to any one of <5> to <7>, in which in the method (1), the hydrophilic group includes at least one selected from the group consisting of a hydroxyl group, a carboxyl group, a sulfo group, and an amino group.

<9> The production method of a polymer according to any one of <5> to <8>, in which in the method (1), the compound (B1) includes at least one selected from the group consisting of 2-mercaptoethanol, 3-mercapto-1,2-propanediol, aminoethanethiol, and sodium 3-mercapto-1-propane sulfonate.

<10> The production method of a polymer according to <5> or <6>, in which in the method (2), the compound (B2) includes at least one selected from the group consisting of ethanol amine, propanol amine, N-(3-aminopropyl) diethanol amine, 3-amino-1,2-propanediol, and diethanol amine.

<11> The production method of a polymer according to <5> or <6>, in which in the method (3), the monomer (B3) includes at least one selected from the group consisting of a hydroxyl group, a carboxyl group, a sulfo group, and an amino group.

<12> The production method of a polymer according to <5>, <6>, or <11>, in which in the method (3), the N-substituted (meth)acrylamide includes at least one selected from the group consisting of hydroxyethyl (meth)acrylamide, hydroxypropyl (meth)acrylamide, and a dimethyl aminopropyl (meth)acrylamide methyl chloride quaternary salt.

<13> The production method of a polymer according to <5>, <6>, <11>, or <12>, in which in the method (3), the sulfonic acid having a vinyl group includes sodium p-styrene sulfonate.

<14> A separation method of a compound, using the polymer according to <3> or <4> for separating a compound.

<15> The separation method of a compound, using a polymer obtained by the production method according to any one of <5> to <13> for separating a compound.

<16> A production method of a compound, including the separation method according to <14> or <15>.

## EFFECT OF THE INVENTION

According to the invention, it is possible to provide a production method of a polymer that can be industrially practically used in which a (meth)acrylic polymer can be simply hydrophilized with a small number of steps.

In addition, according to the invention, it is possible to provide a polymer having excellent hydrophilicity.

Further, according to the invention, it is possible to provide a separating agent that can be preferably used for separating a compound.

MODE(S) FOR CARRYING OUT THE INVENTION

Herein, embodiments of the invention will be described in detail, but the embodiments represent an example of desired embodiments, and the invention is not limited to the contents thereof.

Herein, in the case of using an expression such as "to", "to" is used as an expression including the numerical values or physical property values before and after "to". In addition, herein, "(meth)acryl" indicates "acryl", "methacryl", or both, and "(meth)acrylate" indicates "acrylate", "methacrylate", or both.

[First Embodiment]

[Production Method of Polymer]

A production method according to a first embodiment of the invention is a production method of a polymer (hereinafter, may be referred to as a production method 1), including a method of obtaining a polymer (C1) by reacting a (meth)acrylic polymer (A) (hereinafter, may be referred to as a polymer (A)) with a thiol compound (B1) having a hydrophilic group (hereinafter, may be referred to as a compound (B1)) in accordance with a thiol-ene reaction.

(Polymer (A))

The polymer (A) indicates that a constitutional unit content derived from (meth)acrylate is greater than or equal to 50 mass% in 100 mass% of the total monomer unit configuring the polymer, and the content is preferably greater than or equal to 65 mass%, is more preferably greater than or equal to 80 mass%, and is even more preferably greater than or equal to 95 mass%, from the viewpoint of having excellent hydrophilicity.

Herein, the constitutional unit content derived from (meth)acrylate is a sum total of a constitutional unit content derived from cross-linkable (meth)acrylate described below and a constitutional unit content derived from non-cross-linkable (meth)acrylate described below.

It is preferable that the polymer (A) includes a cross-linked structure from the viewpoint of having excellent reactivity with respect to the compound (B1). In order for the polymer (A) to form the cross-linked structure, it is preferable that the polymer (A) includes a constitutional unit derived from cross-linkable (meth)acrylate.

Examples of cross-linkable (meth)acrylate include di(meth)acrylates such as ethylene glycol di(meth)acrylate, propylene glycol di(meth)acrylate, glycerin di(meth)acrylate, 1,4-butanediol di(meth)acrylate, neopentyl glycol di(meth)acrylate, tetramethylol methane di(meth)acrylate, and hydroquinone di(meth)acrylate; tri(meth)acrylates such as glycerin tri(meth)acrylate, trimethylol propane tri(meth)acrylate, and tetramethylol methane tri(meth)acrylate; tetra(meth)acrylates such as tetramethylol methane tetra(meth)acrylate and dipentaerythritol tetra(meth)acrylate; penta(meth)acrylates such as dipentaerythritol penta(meth)acrylate; and hexa(meth)acrylates such as dipentaerythritol hexa(meth)acrylate. Only one type of such cross-linkable (meth)acrylates may be used, or two or more types thereof may be used together.

In such cross-linkable (meth)acrylates, di(meth)acrylates and tri(meth)acrylates are preferable, ethylene glycol di(meth)acrylate, glycerin di(meth)acrylate, and trimethylol propane tri(meth)acrylate are more preferably, from the viewpoint of being easily industrially produced and of being easily simply available.

The polymer (A) may include a constitutional unit derived from non-cross-linkable (meth)acrylate in addition to the constitutional unit derived from cross-linkable (meth)acrylate.

Examples of non-cross-linkable (meth)acrylate include alkyl (meth)acrylate such as methyl (meth)acrylate, ethyl (meth)acrylate, butyl (meth)acrylate, stearyl (meth)acrylate, 2-ethyl hexyl (meth)acrylate, and cyclohexyl (meth)acrylate; hydroxyl group-containing (meth)acrylate such as hydroxyethyl (meth)acrylate, hydroxypropyl (meth)acrylate, and glycerin mono(meth)acrylate; and epoxy group-containing (meth)acrylate such as glycidyl (meth)acrylate, 4,5-epoxy butyl (meth)acrylate, and 9,10-epoxy stearyl (meth)acrylate. Only one type of such non-cross-linkable (meth)acrylates may be used, or two or more types thereof may be used together.

In such non-cross-linkable (meth)acrylates, alkyl (meth)acrylate and glycidyl (meth)acrylate are preferable from the viewpoint of easily industrially produced and of being easily simply available.

The polymer (A) may include a constitutional unit derived from a monomer other than (meth)acrylate, in addition to the constitutional unit derived from cross-linkable (meth)acrylate and the constitutional unit derived from non-cross-linkable (meth)acrylate.

Examples of the monomer other than (meth)acrylate include styrenes such as styrene, methyl styrene, ethyl styrene, $\alpha$-methyl styrene, chlorostyrene, chloromethyl styrene, and p-styrene sulfonate and alkyl or halogen substitutes

thereof; vinyl esters such as vinyl acetate and vinyl propionate; vinyl ethers such as methyl vinyl ether and ethyl vinyl ether; allyl alcohol and esters or ethers thereof; and (meth)acrylonitrile. Only one type of such monomers other than (meth)acrylate may be used, or two or more types thereof may be used together.

The polymer (A) is obtained by polymerizing a monomer such as cross-linkable (meth)acrylate, non-cross-linkable (meth)acrylate, and the monomer other than (meth)acrylate.

A content rate of cross-linkable (meth)acrylate in the monomer is preferably greater than or equal to 10 mass%, and is more preferably 50 mass% to 100 mass%, in 100 mass% of the total monomer, from the viewpoint of easily forming fine pores and of having an excellent ion exchange adsorption amount and excellent machine characteristics.

A content rate of non-cross-linkable (meth)acrylate in the monomer is preferably less than or equal to 90 mass%, and is more preferably 0 mass% to 50 mass%, in 100 mass% of the total monomer, from the viewpoint of easily forming fine pores and of having an excellent ion exchange adsorption amount and excellent machine characteristics.

A content rate of the monomer other than (meth)acrylate in the monomer is preferably less than or equal to 50 mass%, and is more preferably 0 mass% to 30 mass%, in 100 mass% of the total monomer, from the viewpoint of not impairing the original performance of the polymer (A).

Examples of a polymerization method of the monomer include a solution polymerization method, a suspension polymerization method, and a seed polymerization method. Among such polymerization methods, the suspension polymerization method and the seed polymerization method are preferable from the viewpoint of being capable of obtaining the particulate polymer (C1).

A polymerization condition such as a polymerization temperature, a polymerization time, a polymerization solvent, and a polymerization dispersion medium may be suitably set in accordance with the desired polymer (A) or the desired polymer (C1).

(Compound (B1))

The compound (B1) is a thiol compound having a hydrophilic group.

The hydrophilic group indicates a hydroxyl group or an ion exchange group, and examples thereof include a hydroxyl group; a carboxyl group; a sulfo group; an amino group such as a primary amino group, a secondary amino group, a tertiary amino group, and a quaternary ammonium group; and an acidic functional group such as a phosphate group. Only one type of such hydrophilic groups may be used, or two or more types thereof may be used together.

Among such hydrophilic groups, the hydroxyl group, the carboxyl group, the sulfo group, and the amino group are preferable from the viewpoint of being easily industrially produced and of being easily simply available, and the hydroxyl group is more preferable from the viewpoint of being capable of neutralizing the polymer (C1) and of having excellent handleability.

Examples of the compound (B1) include 2-mercaptoethanol, 3-mercapto-2-propanol, 3-mercapto-2-butanol, 3-mercapto-1,2-propanediol, a thioglycolic acid, cysteine, aminoethanethiol, 1-aminopropane-2-thiol, sodium 2-mercaptoethane sulfonate, and sodium 3-mercapto-1-propane sulfonate. Only one type of such compounds (B1) may be used, or two or more types thereof may be used together.

Among such compounds (B1), 2-mercaptoethanol, 3-mercapto-1,2-propanediol, aminoethanethiol, and sodium 3-mercapto-1-propane sulfonate are preferable from the viewpoint of being easily industrially produced and of being easily simply available, and 3-mercapto-1,2-propanediol is more preferable from the viewpoint of being capable of neutralizing the polymer (C1) and of having excellent handleability.

(Reaction between Polymer (A) and Compound (B1))

The reaction between the polymer (A) and the compound (B1) is a reaction between a double bond of the polymer (A) and thiol of the compound (B1) (the thiol-ene reaction).

The double bond of the polymer (A) can be introduced to the polymer (A) by using cross-linkable (meth)acrylate as a monomer in the polymerization for obtaining the polymer (A). In order to increase an introduction amount of the double bond of the polymer (A), a content rate of cross-linkable (meth)acrylate in the monomer that is used in the polymerization for obtaining the polymer (A) may be increased.

The reaction between the double bond of the polymer (A) and thiol of the compound (B1) may be a radical addition reaction, or may be an anionic addition reaction, and the radical addition reaction is preferable from the viewpoint of having excellent reactivity.

In a case where the reaction between the double bond of the polymer (A) and thiol of the compound (B1) is the radical addition reaction, the reaction is started by adding a radical generating agent.

Examples of the radical generating agent include a peroxide-based thermal radical generating agent such as tert-butyl hydroperoxide, cumene hydroperoxide, peroxyacetate, a peracetic acid, a chloroperbenzoic acid, ammonium persulfate, sodium persulfate, and potassium peroxodisulfate; an azo-based thermal radical generating agent such as

a 4,4'-azobis(4-cynovaleric acid), 2,2'-azobis(2-methyl propione amidine) dihydrochloride, and a 2,2'-azobis[N-(2-carboxyethyl)-2-methyl propione amidine] n-hydrate; and a photoradical generating agent such as 1-[4-(2-hydroxyethoxy)-phenyl]-2-hydroxy-2-methyl propane. Only one type of such radical generating agents may be used, or two or more types thereof may be used together.

Among such radical generating agents, a radical generating agent in which the compound (B1) is dissolved in a solvent described below is preferable, the azo-based thermal radical generating agent is more preferable, and 2,2'-azobis(2-methyl propione amidine) dihydrochloride and a 2,2'-azobis[N-(2-carboxyethyl)-2-methyl propione amidine] n-hydrate are even more preferable, from the viewpoint of having excellent reactivity.

In a case where the reaction between the double bond of the polymer (A) and thiol of the compound (B1) is the anionic addition reaction, the reaction is started by adding a basic compound.

Examples of the basic compound include an inorganic basic compound such as a metal hydroxide and a metal carbonate compound; and an organic basic compound such as organic amine. Only one type of such basic compounds may be used, or two or more types thereof may be used together. Among such basic compounds, the inorganic basic compound is preferable, and a metal hydroxide is more preferable, from the viewpoint of having excellent reactivity.

An additive amount of the compound (B1) with respect to the polymer (A) is preferably 10 parts by mass to 300 parts by mass, and is more preferably 50 parts by mass to 200 parts by mass, with respect to 100 parts by mass of the polymer (A). In a case where the additive amount of the compound (B1) with respect to the polymer (A) is greater than or equal to 10 parts by mass, the amount of hydrophilic group in the polymer (C1) increases, and the polymer (C1) has excellent hydrophilicity. In addition, in a case where the additive amount of the compound (B1) with respect to the polymer (A) is less than or equal to 300 parts by mass, the amount of compound (B1) to be unreacted can be suppressed.

In the reaction between the polymer (A) and the compound (B1), the solvent may be used, or the solvent may not be used, but it is preferable to use the solvent from the viewpoint of being capable of homogeneously dispersing the polymer (A) and the compound (B1).

Examples of the solvent include water; ethers such as diethyl ether, tetrahydrofuran, and dioxane; hydrocarbons such as toluene and xylene; halogenated hydrocarbons such as halobenzene, dichloromethane, dichloroethane, and chloroform; alcohols such as methanol, ethanol, and isopropanol; and nitriles such as acetonitrile; polar solvents such as dimethyl formamide, dimethyl acetamide, dimethyl sulfoxide, and N-methyl-2-pyrrolidone. Only one type of such solvents may be used, or two or more types thereof may be used together. Among such solvents, the solvent in which the compound (B1) is dissolved is preferable, and water is more preferable, from the viewpoint of having excellent reactivity.

A reaction temperature of the polymer (A) and the compound (B1) is preferably 0°C to 300°C, and is more preferably 10°C to 200°C, from the viewpoint of having excellent reactivity.

A reaction atmosphere of the polymer (A) and the compound (B1) is not particularly limited, and may be an air atmosphere, or may be an inert gas atmosphere.

A reaction time of the polymer (A) and the compound (B1) is preferably 1 hour to 30 hours, and is more preferably 2 hours to 10 hours, from the viewpoint of sufficient progress of the reaction.

A purification step such as solvent distillation, filtration, and washing may be provided after the reaction between the polymer (A) and the compound (B1).

In addition, the polymer (C1) may be obtained through an oxidation step after the reaction between the polymer (A) and the compound (B1). Sulfoxide or sulfone is formed through the oxidation step, and thus, the polymer (C1) has more excellent hydrophilicity.

Examples of an oxidation method include a method of reacting the polymer obtained by the reaction between the polymer (A) and the compound (B1) with an oxidant.

Examples of the oxidant include sodium periodate, sodium hypochlorite, hydrogen peroxide, meta-chlorobenzoic acid, and potassium hydrogen persulfate. Only one type of such oxidants may be used, or two or more types thereof may be used together. Among such oxidants, sodium periodate and sodium hypochlorite are preferable, and sodium hypochlorite is more preferable, from the viewpoint of suppressing excessive oxidation and of having excellent reactivity of the polymer obtained by the reaction between the polymer (A) and the compound (B1).

As described above, the polymer (C1) (the hydrophilized (meth)acrylic polymer) is obtained by the reaction between the polymer (A) and the compound (B1).

[Polymer (C1)]

The polymer (C1) includes a structure represented by General Formula (3) described below.

[Chemical Formula 5]

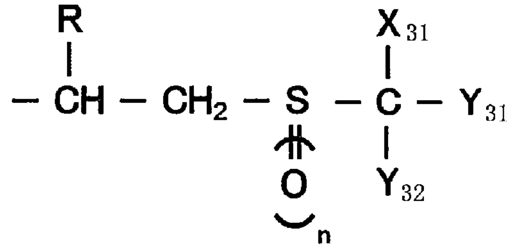

(3)

(In General Formula (3) described above, $X_{31}$ represents a hydrophilic group-containing structure, $Y_{31}$ and $Y_{32}$ each independently represent a hydrophilic group-containing structure, a hydrogen atom, or an alkyl group, n represents an integer of 0 to 2, and R represents a hydrogen atom or an alkyl group.)

$X_{31}$ represents a hydrophilic group-containing structure.

The hydrophilic group indicates a hydroxyl group or an ion exchange group, and examples thereof include a hydroxyl group; a carboxyl group; a sulfo group; an amino group such as a primary amino group, a secondary amino group, a tertiary amino group, and a quaternary ammonium group; and an acidic functional group such as a phosphate group. Only one type of such hydrophilic groups may be used, or two or more types thereof may be used together.

Among such hydrophilic groups, the hydroxyl group, the carboxyl group, the sulfo group, and the amino group are preferable from the viewpoint of being easily industrially produced and of being easily simply available.

An alkyl group can be included in the hydrophilic group-containing structure in $X_{31}$.

Examples of the alkyl group include a linear or branched alkyl group having 1 to 4 carbon atoms.

Herein, examples of the linear or branched alkyl group having 1 to 4 carbon atoms include a methyl group, an ethyl group, an n-propyl group, an isopropyl group, an n-butyl group, an isobutyl group, a sec-butyl group, and a tert-butyl group.

Among such $X_{31}$, a hydrophilic group and an alkyl group-containing structure are preferable, 1 to 3 hydrophilic groups and an alkyl group-containing structure having 1 to 4 carbon atoms are more preferable, and one to two hydrophilic groups and an alkyl group-containing structure having 1 to 2 carbon atoms are even more preferable, from the viewpoint of being easily industrially produced and of having excellent hydrophilicity.

Specific examples of the hydrophilic group and the alkyl group-containing structure include $-CH_2OH$, $-CHOHCH_2OH$, $-CH_2NH_2$, $-C_2H_4SO_3Na$, $-CH_2COOH$, and $-CNH_2COOH$. Among such structures, $-CH_2OH$, $-CHOHCH_2OH$, $-CH_2NH_2$, and $-C_2H_4SO_3Na$ are preferable, and $-CHOHCH_2OH$ is more preferable, from the viewpoint of being easily industrially produced and of having excellent hydrophilicity.

The same structure as the hydrophilic group-containing structure in $X_{31}$ can be used as the hydrophilic group-containing structure in $Y_{31}$ and $Y_{32}$.

Examples of the alkyl group in $Y_{31}$ and $Y_{32}$ include a linear or branched alkyl group having 1 to 4 carbon atoms.

Among such $Y_{31}$ and $Y_{32}$, a hydrophilic group-containing structure and a hydrogen atom are preferable, and a hydrogen atom is more preferable, from the viewpoint of being easily industrially produced and of having excellent hydrophilicity.

n represents an integer of 0 to 2. In the case of performing the oxidation step after the reaction between the polymer (A) and the compound (B1), n is 0, and in the case of not performing the oxidation step, n is a mixture of 0, 1, and 2.

Examples of the alkyl group in R include a linear or branched alkyl group having 1 to 4 carbon atoms.

Among such R, a hydrogen atom and an alkyl group having 1 to 2 carbon atoms are preferable, and a hydrogen atom and an alkyl group having one carbon atom are more preferable, from the viewpoint of being easily industrially produced.

In addition, the structure represented by General Formula (3) is a structure represented by General Formula (3a) described below.

[Chemical Formula 6]

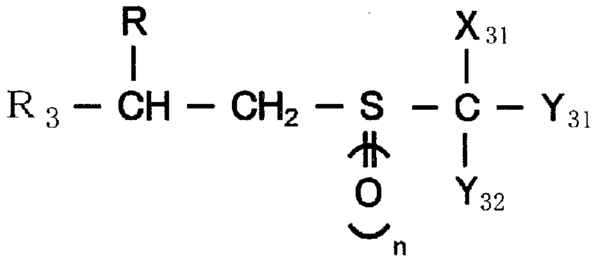

(3a)

The definition of $X_{31}$, $Y_{31}$, $Y_{32}$, n, and R in General Formula (3a) described above is the same as the definition of $X_{31}$, $Y_{31}$, $Y_{32}$, n, and R in General Formula (3) described above.

It is preferable that $R_3$ in General Formula (3a) described above is a (meth)acrylic polymer from the viewpoint of being capable of easily introducing a sulfide structure.

In addition, it is preferable that the structure represented by General Formula (3a) is a structure represented by General Formula (3b) described below.

[Chemical Formula 7]

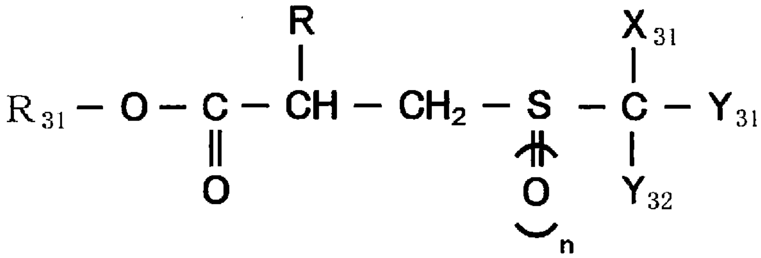

(3b)

The definition of $X_{31}$, $Y_{31}$, $Y_{32}$, n, and R in General Formula (3b) described above is the same as the definition of $X_{31}$, $Y_{31}$, $Y_{32}$, n, and R in General Formula (3) described above.

It is preferable that $R_{31}$ in General Formula (3b) described above is a (meth)acrylic polymer from the viewpoint of being capable of easily introducing the sulfide structure.

The shape of the polymer (C1) may be suitably set as usage, and examples thereof include a particulate shape, a film shape, and a plate shape.

The polymer (C1) may be porous, or may be nonporous. In a case where the polymer (C1) is porous, a pore forming agent may be used at the time of obtaining the polymer (A).

The pore forming agent functions as a phase separating agent in the polymerization of a monomer such as (meth)acrylate at the time of obtaining the polymer (A), dissolves the monomer such as (meth)acrylate with an organic solvent that accelerates the formation of the pores, but it is preferable that the pore forming agent does not dissolve the polymer (A).

Specifically, specific examples of the pore forming agent include aliphatic or alicyclic hydrocarbons such as hexane, heptane, octane, dodecane, and cyclohexane; aromatic hydrocarbons such as benzene, toluene, xylene, and ethyl benzene; ketones such as methyl ethyl ketone and 4-methyl-2-pentanone; ethers such as dibutyl ether; aliphatic or alicyclic alcohols such as hexanol, octanol, dodecanol, cyclohexanol, and lauryl alcohol; esters such as ethyl acetate, butyl acetate, dimethyl phthalate, and diethyl phthalate; halogenated hydrocarbons such as dichloromethane, dichloroethane, and trichloroethylene; and aromatic halogenated hydrocarbons such as chlorobenzene and dichlorobenzene. Only one type of such pore forming agents may be used, or two or more types thereof may be used together. Among such pore forming agents, aliphatic or alicyclic hydrocarbons, aromatic hydrocarbons, and ketones are preferable from the viewpoint of easily forming desired fine pores.

The polymer (C1) has excellent hydrophilicity, and thus, can be preferably used in a usage in which hydrophilicity is necessary, for example, can be preferably used in compound separating particles, a compound separating film, an antifouling resin plate, an antifogging resin plate, an antistatic plate, a resin modifier, and the like, and can be particularly preferably used for separating a compound.

The polymer (C1) may be further subjected to a treatment such as the modification of a functional group before being used as the usage.

In the case of using the polymer (C1) for separating a compound, porous particles and a porous film are

preferable as the shape of the polymer (C1) from the viewpoint of having excellent separation capacity, and the porous particles are more preferable as the shape of the polymer (C1) from the viewpoint of being capable of easily separating a compound by filling a liquid chromatography column with the compound.

A modal fine pore radius of the porous particles is preferably 10 angstroms to 2000 angstroms, and is more preferably 50 angstroms to 500 angstroms. In a case where the modal fine pore radius of the porous particles is greater than or equal to 10 angstroms, the diffusivity of an adsorption target substance is excellent, and an adsorption amount is excellent. In addition, in a case where the modal fine pore radius of the porous particles is less than or equal to 2000 angstroms, the strength of the porous particles is excellent.

Herein, the modal fine pore radius of the porous particles is measured by a nitrogen gas adsorption method. Specifically, the modal fine pore radius of the porous particles is calculated from a pressure and an adsorption amount when nitrogen gas molecules are condensed in the fine pores.

A fine pore volume of the porous particles is preferably 0.4 mL/g to 1.5 mL/g, and is more preferably 0.7 mL/g to 1.2 mL/g. In a case where the fine pore volume of the porous particles is greater than or equal to 0.4 mL/g, the diffusivity of the adsorption target substance is excellent, and the adsorption amount is excellent. In addition, in a case where the fine pore volume of the porous particles is less than or equal to 1.5 mL/g, the strength of the porous particles is excellent.

Herein, the fine pore volume of the porous particles is measured by a nitrogen gas adsorption method. Specifically, the fine pore volume of the porous particles is calculated from a pressure and an adsorption amount when nitrogen gas molecules are condensed in the fine pores.

A specific surface area of the porous particles is preferably 30 $m^2/g$ to 700 $m^2/g$, and is more preferably 100 $m^2/g$ to 600 $m^2/g$. In a case where the specific surface area of the porous particles is greater than or equal to 30 $m^2/g$, many hydroxyl groups can be introduced, and the adsorption amount is excellent. In addition, in a case where the specific surface area of the porous particles is less than or equal to 700 $m^2/g$, it does not take time until the adsorption target substance reaches the fine pores, and a dynamic adsorption amount is excellent.

Herein, the specific surface area of the porous particles is measured by a nitrogen gas adsorption method (a BET method). Specifically, a monomolecular layer adsorption amount is calculated by the BET method from a pressure change before and after the adsorption of nitrogen gas, and the specific surface area of the porous particles is calculated from a sectional area of one molecule of the nitrogen gas, to which ISO 9277 is applied.

A volume average particle diameter of the porous particles is preferably 1 μm to 1000 μm, is more preferably 5 μm to 700 μm, and is even more preferably 10 μm to 500 μm. In a case where the volume average particle diameter of the porous particles is greater than or equal to 1 μm, a pressure loss when a column is filled with the porous particles and liquid passing is performed is suppressed, a liquid passing velocity can be increased,
and the productivity of a separating treatment is excellent. In addition, in a case where the volume average particle diameter of the porous particles is less than or equal to 1000 μm, a column efficiency is excellent, and the adsorption amount or the separation capacity is excellent.

Herein, the volume average particle diameter of the porous particles is obtained by measuring particle diameters of arbitrary 400 porous particles with an optical microscope and by calculating a volume median size from the distribution thereof.

An average pore diameter of the porous film is preferably 1 nm to 50 nm, and is more preferably 2 nm to 40 nm. In a case where the average pore diameter of the porous film is greater than or equal to 1 nm, liquid passing properties are excellent. In addition, in a case where the average pore diameter of the porous film is less than or equal to 50 nm, the separation capacity is excellent.

[Separating Agent]

The compound hydrophilized in the production method 1 can be used as a separating agent (hereinafter, may be referred to as a separating agent 1). The separating agent 1 includes a structure represented by General Formula (1) described below.

[Chemical Formula 8]

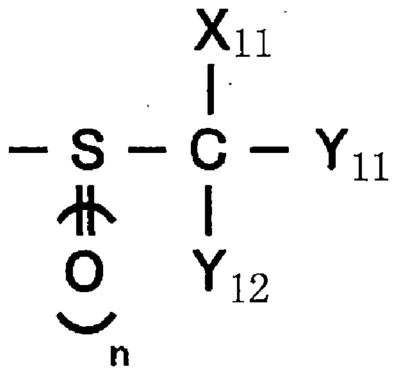

(1)

(In General Formula (1) described above, $X_{11}$ represents a hydrophilic group-containing structure, $Y_{11}$ and $Y_{12}$ each independently represent a hydrophilic group-containing structure, a hydrogen atom, or an alkyl group, and n represents an integer of 0 to 2.)

$X_{11}$ represents a hydrophilic group-containing structure.

The hydrophilic group indicates a hydroxyl group or an ion exchange group, and examples thereof include a hydroxyl group; a carboxyl group; a sulfo group; an amino group such as a primary amino group, a secondary amino group, a tertiary amino group, and a quaternary ammonium group; and an acidic functional group such as a phosphate group. Only one type of such hydrophilic groups may be used, or two or more types thereof may be used together.

Among such hydrophilic groups, the hydroxyl group, the carboxyl group, the sulfo group, and the amino group are preferable from the viewpoint of being easily industrially produced and of being easily simply available.

An alkyl group can be included in the hydrophilic group-containing structure in $X_{11}$.

Examples of the alkyl group include a linear or branched alkyl group having 1 to 4 carbon atoms.

Among such $X_{11}$, a hydrophilic group and an alkyl group-containing structure are preferable, 1 to 3 hydrophilic groups and an alkyl group-containing structure having 1 to 4 carbon atoms are more preferably, and 1 to 2 hydrophilic groups and an alkyl group-containing structure having 1 to 2 carbon atoms are even more preferable, from the viewpoint of being easily industrially produced and of having excellent hydrophilicity.

Specific examples of the hydrophilic group and the alkyl group-containing structure include $-CH_2OH$, $-CHOHCH_2OH$, $-CH_2NH_2$, $-C_2H_4SO_3Na$, $-CH_2COOH$, and $-CNH_2COOH$. Among such structures, $-CH_2OH$, $-CHOHCH_2OH$, $-CH_2NH_2$, and $-C_2H_4SO_3Na$ are preferable, and $-CHOHCH_2OH$ is more preferable, from the viewpoint of being easily industrially produced and of having excellent hydrophilicity.

The same structure as the hydrophilic group-containing structure in $X_{11}$ can be used as the hydrophilic group-containing structure in $Y_{11}$ and $Y_{12}$.

Examples of the alkyl group in $Y_{11}$ and $Y_{12}$, include a linear or branched alkyl group having 1 to 4 carbon atoms.

Among such $Y_{11}$ and $Y_{11}$, a hydrophilic group-containing structure and a hydrogen atom are preferable, and a hydrogen atom is more preferable, from the viewpoint of being easily industrially produced and of having excellent hydrophilicity.

n represents an integer of 0 to 2. In the case of performing the oxidation step after the reaction between the polymer (A) and the compound (B1), n is 0, and in the case of not performing the oxidation step, n is a mixture of 0, 1, and 2.

In addition, the structure represented by General Formula (1) is preferably the structure represented by General Formula (3) described above, is more preferably the structure represented by General Formula (3a) described above, and is even more preferably the structure represented by General Formula (3b) described above.

In addition, the separating agent 1, for example, can be obtained by the production method 1 described above.

A preferred shape of the separating agent 1 is the same as a preferred shape of the polymer (C1).

[Second Embodiment]

[Production Method of Polymer]

A production method according to a second embodiment of the invention is a production method of a polymer (hereinafter, may be referred to as a production method 2), including a method of obtaining a polymer (C2) by reacting the (meth)acrylic polymer (A) (hereinafter, may be referred to as the polymer (A)) with an aminoalcohol compound (B2) (hereinafter, may be referred to as a compound (B2)) in accordance with an ester exchange reaction.

(Polymer (A))

In the second embodiment of the invention, the same polymer as the polymer (A) that is used in the first embodiment of the invention can be used.

(Compound (B2))

The compound (B2) is an aminoalcohol compound.

Examples of the compound (B2) include ethanol amine, N-methyl ethanol amine, diethanol amine, propanol amine, alaninol, N-(3-aminopropyl) diethanol amine, 3-amino-1,2-propanediol, and 3-methyl amino-1,2-propanediol. Only one type of such compounds (B2) may be used, or two or more types thereof may be used together.

Among such compounds (B2), ethanol amine, propanol amine, N-(3-aminopropyl) diethanol amine, 3-amino-1,2-propanediol, and diethanol amine are preferable, ethanol amine, propanol amine, and diethanol amine are more preferable, and ethanol amine and diethanol amine are even more preferable, from the viewpoint of having excellent reactivity with respect to the polymer (A).

(Reaction between Polymer (A) and Compound (B2))

The reaction between the polymer (A) and the compound (B2) is a reaction between an ester bond of the polymer (A) and amine of the compound (B2) (the ester exchange reaction).

The ester bond of the polymer (A) can be introduced to the polymer (A) by using (meth)acrylate as a monomer in the polymerization for obtaining the polymer (A). In order to increase an introduction amount of the ester bond of the polymer (A), a content rate of (meth)acrylate in the monomer that is used in the polymerization for obtaining the polymer (A) may be increased.

An additive amount of the compound (B2) with respect to the polymer (A) is preferably 10 parts by mass to 3000 parts by mass, and is more preferably 100 parts by mass to 2000 parts by mass, with respect to 100 parts by mass of the polymer (A). In a case where the additive amount of the compound (B2) with respect to the polymer (A) is greater than or equal to 10 parts by mass, reactivity is excellent. In addition, in a case where the additive amount of the compound (B2) with respect to the polymer (A) is less than or equal to 3000 parts by mass, handleability and an economic efficiency are excellent.

In the reaction between the polymer (A) and the compound (B2), a basic compound may be added as necessary in order to start and accelerate the reaction.

Examples of the basic compound include an inorganic basic compound such as a metal hydroxide and a metal carbonate compound; and an organic basic compound such as organic amine. Only one type of such basic compounds may be used, or two or more types thereof may be used together. Among such basic compounds, the organic basic compound is preferable from the viewpoint of having excellent reactivity.

In the reaction between the polymer (A) and the compound (B2), the solvent may be used, or the solvent may not be used, but it is preferable to use the solvent from the viewpoint of being capable of homogeneously dispersing the polymer (A) and the compound (B2).

Examples of the solvent include water; ethers such as diethyl ether, tetrahydrofuran, and dioxane; hydrocarbons such as toluene and xylene; halogenated hydrocarbons such as halobenzene, dichloromethane, dichloroethane, and chloroform; alcohols such as methanol, ethanol, and isopropanol; nitriles such as acetonitrile; and polar solvents such as dimethyl formamide, dimethyl acetamide, dimethyl sulfoxide, and N-methyl-2-pyrrolidone. Only one type of such solvents may be used, or two or more types thereof may be used together. Among such solvents, a solvent in which the compound (B2) is dissolved is preferable, water, tetrahydrofuran, dioxane, and ethanol are more preferable, and water and tetrahydrofuran are even more preferable, from the viewpoint of having excellent reactivity.

A reaction temperature of the polymer (A) and the compound (B2) is preferably 0°C to 300°C, and is more preferably 10°C to 200°C, from the viewpoint of having excellent reactivity.

A reaction atmosphere of the polymer (A) and the compound (B2) is not particularly limited, and may be an air atmosphere, or may be an inert gas atmosphere.

A reaction time of the polymer (A) and the compound (B2) is preferably 1 hour to 30 hours, and is more preferably 2 hours to 10 hours, from the viewpoint of sufficient progress of the reaction.

A purification step such as solvent distillation, filtration, and washing may be provided after the reaction between the polymer (A) and the compound (B2).

As described above, the polymer (C2) (the hydrophilized (meth)acrylic polymer) is obtained by the reaction between the polymer (A) and the compound (B2).

[Polymer (C2)]

The polymer (C2) includes a structure represented by General Formula (4) described below.

[Chemical Formula 9]

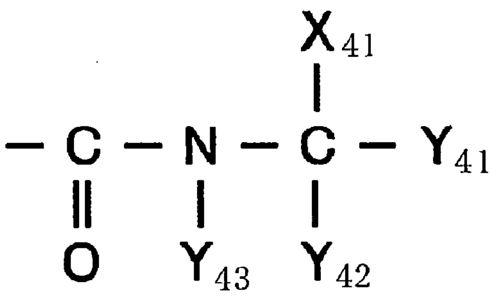

(4)

(In General Formula (4) described above, $X_{41}$ represents a hydrophilic group-containing structure, and $Y_{41}$ to $Y_{43}$ each independently represent a hydrophilic group-containing structure, a hydrogen atom, or an alkyl group.)

$X_{41}$ represents a hydrophilic group-containing structure.

The hydrophilic group indicates a hydroxyl group or an ion exchange group, and examples thereof include a hydroxyl group; a carboxyl group; a sulfo group; an amino group such as a primary amino group, a secondary amino group, a tertiary amino group, and a quaternary ammonium group; and an acidic functional group such as a phosphate group. Only one type of such hydrophilic groups may be used, or two or more types thereof may be used together.

Among such hydrophilic groups, the hydroxyl group, the carboxyl group, the sulfo group, and the amino group are preferable from the viewpoint of being easily industrially produced and of being easily simply available.

An alkyl group can be included in the hydrophilic group-containing structure in $X_{41}$.

Examples of the alkyl group include a linear or branched alkyl group having 1 to 4 carbon atoms.

Among such $X_{41}$, a hydrophilic group and an alkyl group-containing structure are preferable, 1 to 3 hydrophilic groups and an alkyl group-containing structure having 1 to 4 carbon atoms are more preferable, and 1 to 2 hydrophilic groups and an alkyl group-containing structure having 1 to 2 carbon atoms are even more preferable, from the viewpoint of being easily industrially produced and of having excellent hydrophilicity.

Specific examples of the hydrophilic group and the alkyl group-containing structure include $-CH_2OH$, $-CHOHCH_2OH$, $-CH_2NH_2$, $-C_2H_4SO_3Na$, $-CH_2COOH$, and $-CNH_2COOH$. Among such structures, $-CH_2OH$, $-CHOHCH_2OH$, $-CH_2NH_2$, and $-C_2H_4SO_3Na$ are preferable, and $-CH_2OH$ is more preferable, from the viewpoint of being easily industrially produced and of having excellent hydrophilicity.

The same structure as the hydrophilic group-containing structure in $X_{41}$ can be used as the hydrophilic group-containing structure in $Y_{41}$ to $Y_{43}$.

Examples of the alkyl group in $Y_{41}$ to $Y_{43}$ include a linear or branched alkyl group having 1 to 4 carbon atoms.

Among such $Y_{41}$ to $Y_{43}$, a hydrophilic group-containing structure and a hydrogen atom are preferable, and a hydrogen atom is more preferable, from the viewpoint of being easily industrially produced and of having excellent hydrophilicity.

In addition, it is preferable that the structure represented by General Formula (4) is a structure represented by General Formula (4a) described below.

[Chemical Formula 10]

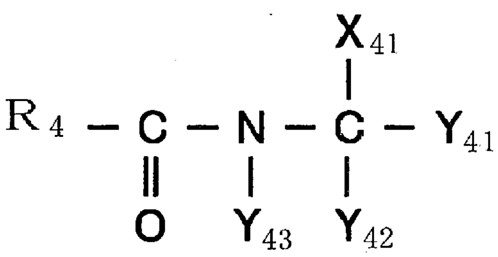

(4a)

The definition of $X_{41}$ and $Y_{41}$ to $Y_{43}$ in General Formula (4a) described above is the same as the definition of $X_{41}$ and $Y_{41}$ to $Y_{43}$ in General Formula (4) described above.

It is preferable that $R_4$ in General Formula (4a) described above is a (meth)acrylic polymer from the viewpoint

of being capable of easily introducing an amide structure.

In addition, it is preferable that the structure represented by General Formula (4a) is a structure represented by General Formula (4b) described below.

[Chemical Formula 11]

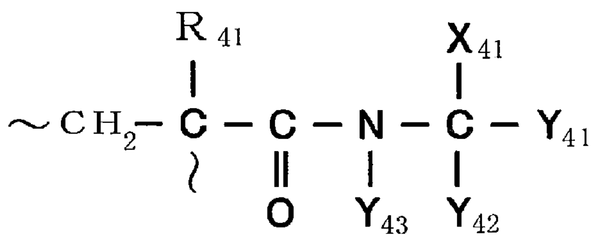

(4b)

The definition of $X_{41}$ and $Y_{41}$ to $Y_{43}$ in General Formula (4b) described above is the same as the definition of $X_{41}$ and $Y_{41}$ to $Y_{43}$ in General Formula (4) described above.

It is preferable that a polymer chain of the (meth)acrylic polymer is bonded to "~" in General Formula (4b) described above.

$R_{41}$ in General Formula (4b) described above represents a hydrogen atom or an alkyl group. Examples of the alkyl group include a linear or branched alkyl group having 1 to 4 carbon atoms.

Among such $R_{41}$, a hydrogen atom and an alkyl group having 1 to 2 carbon atoms are preferable, and a hydrogen atom and an alkyl group having one carbon atom are more preferable, from the viewpoint of being easily industrially produced.

A preferred shape of the polymer (C2) is the same as the preferred shape of the polymer (C1).

The polymer (C2) has excellent hydrophilicity, and thus, can be preferably used in a usage in which hydrophilicity is necessary, for example, can be preferably used in compound separating particles, a compound separating film, an antifouling resin plate, an antifogging resin plate, an antistatic plate, a resin modifier, and the like, and can be particularly preferably used for separating a compound.

The polymer (C2) may be further subjected to a treatment such as the modification of a functional group before being used as the usage.

In the case of using the polymer (C2) for separating a compound, porous particles and a porous film are preferable as the shape of the polymer (C2) from the viewpoint of having excellent separation capacity, and the porous particles are more preferable as the shape of the polymer (C2) from the viewpoint of being capable of easily separating a compound by filling a liquid chromatography column with the compound.

Preferred values of a modal fine pore radius of the porous particles, a fine pore volume, a specific surface area, a volume average particle diameter, and an average pore diameter of the porous film are the same as those in the case of the polymer (C1).

[Separating Agent]

The compound hydrophilized in the production method 2 can be used as a separating agent (hereinafter, may be referred to as a separating agent 2). The separating agent 2 includes a structure represented by General Formula (2) described below.

[Chemical Formula 12]

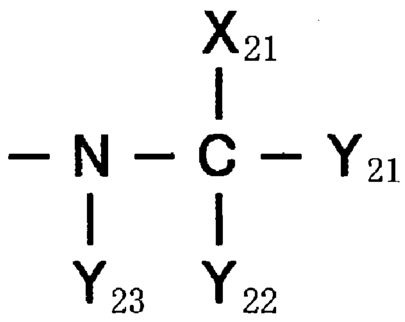

(2)

(In General Formula (2) described above, $X_{21}$ represents a hydrophilic group-containing structure, and $Y_{21}$ to $Y_{23}$ each independently represent a hydrophilic group-containing structure, a hydrogen atom, or an alkyl group.)

$X_{21}$ represents a hydrophilic group-containing structure.

The hydrophilic group indicates a hydroxyl group or an ion exchange group, and examples thereof include a hydroxyl group; a carboxyl group; a sulfo group; an amino group such as a primary amino group, a secondary amino group, a tertiary amino group, and a quaternary ammonium group; and an acidic functional group such as a phosphate group. Only one type of such hydrophilic groups may be used, or two or more types thereof may be used together.

Among such hydrophilic groups, the hydroxyl group, the carboxyl group, the sulfo group, and the amino group are preferable from the viewpoint of being easily industrially produced and of being easily simply available.

An alkyl group can be included in the hydrophilic group-containing structure in $X_{21}$.

Examples of the alkyl group include a linear or branched alkyl group having 1 to 4 carbon atoms.

Among such $X_{21}$, a hydrophilic group and an alkyl group-containing structure are preferable, 1 to 3 hydrophilic groups and an alkyl group-containing structure having 1 to 4 carbon atoms are more preferable, and 1 to 2 hydrophilic groups and an alkyl group-containing structure having 1 to 2 carbon atoms are even more preferable, from the viewpoint of being easily industrially produced and of having excellent hydrophilicity.

Specific examples of the hydrophilic group and the alkyl group-containing structure include $-CH_2OH$, $-CHOHCH_2OH$, $-CH_2NH_2$, $-C_2H_4SO_3Na$, $-CH_2COOH$, and $-CNH_2COOH$. Among such structures, $-CH_2OH$, $-CHOHCH_2OH$, $-CH_2NH_2$, and $-C_2H_4SO_3Na$ are preferable, and $-CH_2OH$ is more preferable, from the viewpoint of being easily industrially produced and of having excellent hydrophilicity.

The same structure as the hydrophilic group-containing structure in $X_{21}$ can be used as the hydrophilic group-containing structure in $Y_{21}$ to $Y_{23}$.

Examples of the alkyl group in $Y_{21}$ to $Y_{23}$ include a linear or branched alkyl group having 1 to 4 carbon atoms.

Among such $Y_{21}$ to $Y_{23}$, a hydrophilic group-containing structure and a hydrogen atom are preferable, and a hydrogen atom is more preferable, from the viewpoint of being easily industrially produced and of having excellent hydrophilicity.

In addition, the structure represented by General Formula (2) is preferably the structure represented by General Formula (4) described above, is more preferably the structure represented by General Formula (4a) described above, and is even more preferably the structure represented by General Formula (4b) described above.

In addition, the separating agent 2, for example, can be obtained by the production method 2 described above.

A preferred shape of the separating agent 2 is the same as the preferred shape of the polymer (C1).

[Third Embodiment]

[Production Method of Polymer]

A production method according to a third embodiment of the invention is a production method of a polymer (hereinafter, may be referred to as a production method 3), including a method of obtaining a polymer (C3) by polymerizing a monomer (B3) including at least one selected from the group consisting of a sulfonic acid having a vinyl group and N-substituted (meth)acrylamide in the presence of the (meth)acrylic polymer (A) (hereinafter, may be referred to as the polymer (A)).

(Polymer (A))

In the third embodiment of the invention, the same polymer as the polymer (A) that is used in the first embodiment

of the invention can be used.

(Monomer (B3))

The monomer (B3) includes at least one selected from the group consisting of a sulfonic acid having a vinyl group and N-substituted (meth)acrylamide.
Among the monomers (B3), it is preferable to include a sulfonic acid having a vinyl group in the case of planning to perform cation exchange with respect to the polymer (C3).
Among the monomers (B3), it is preferable to include N-substituted (meth)acrylamide in the case of not allowing the polymer (C3) to have an ion exchange group or of planning to perform anion exchange with respect to the polymer (C3).
It is preferable that the monomer (B3) includes a hydrophilic group from the viewpoint of hydrophilizing the polymer (C3).
The hydrophilic group indicates a hydroxyl group or an ion exchange group, and examples thereof include a hydroxyl group; a carboxyl group; a sulfo group; an amino group such as a primary amino group, a secondary amino group, a tertiary amino group, and a quaternary ammonium group; and an acidic functional group such as a phosphate group. Only one type of such hydrophilic groups may be used, or two or more types thereof may be used together.
Among such hydrophilic groups, the hydroxyl group, the carboxyl group, the sulfo group, and the amino group are preferable from the viewpoint of being easily industrially produced and of being easily simply available, and the hydroxyl group, the sulfo group, and the amino group are more preferable from the viewpoint of being capable of neutralizing the polymer (C3) and of having excellent handleability.
Examples of a sulfonic acid having a vinyl group include a vinyl sulfonic acid, sodium vinyl sulfonate, and sodium p-styrene sulfonate. Only one type of such sulfonic acids having a vinyl group may be used, or two or more types thereof may be used together.
Among such sulfonic acids having a vinyl group, sodium vinyl sulfonate and sodium p-styrene sulfonate are preferable, and sodium p-styrene sulfonate is more preferable, from the viewpoint of having excellent solubility with respect to water.
Examples of N-substituted (meth)acrylamide include hydroxyethyl (meth)acrylamide, hydroxypropyl (meth)acrylamide, a dimethyl aminopropyl (meth)acrylamide methyl chloride quaternary salt, and dimethyl aminopropyl (meth)acrylamide. Only one type of such N-substituted (meth)acrylamides may be used, or two or more types thereof may be used together.
Among such N-substituted (meth)acrylamides, hydroxyethyl (meth)acrylamide, hydroxypropyl (meth)acrylamide, and a dimethyl aminopropyl (meth)acrylamide methyl chloride quaternary salt are preferable, and hydroxyethyl (meth)acrylamide and a dimethyl aminopropyl (meth)acrylamide methyl chloride quaternary salt are more preferable, from the viewpoint of excellent hydrophilicity of the polymer (C3).
The monomer (B3) may include other monomers in addition to a sulfonic acid having a vinyl group and N-substituted (meth)acrylamide.
Examples of the other monomers include alkyl (meth)acrylate such as methyl (meth)acrylate, ethyl (meth)acrylate, butyl (meth)acrylate, stearyl (meth)acrylate, 2-ethyl hexyl (meth)acrylate, and cyclohexyl (meth)acrylate; hydroxyl group-containing (meth)acrylate such as hydroxyethyl (meth)acrylate, hydroxypropyl (meth)acrylate, and glycerin mono(meth)acrylate; epoxy group-containing (meth)acrylate such as glycidyl (meth)acrylate, 4,5-epoxy butyl (meth)acrylate, and 9,10-epoxy stearyl (meth)acrylate; styrenes such as styrene, methyl styrene, ethyl styrene, $\alpha$-methyl styrene, chlorostyrene, and chloromethyl styrene and alkyl or halogen substitutes thereof; vinyl esters such as vinyl acetate and vinyl propionate; vinyl ethers such as methyl vinyl ether and ethyl vinyl ether; allyl alcohol and ester or ethers thereof; and (meth)acrylonitriles. Only one type of such other monomers may be used, or two or more types thereof may be used together.
A content rate of a sum total of a sulfonic acid having a vinyl group and N-substituted (meth)acrylamide in the monomer (B3) is preferably greater than or equal to 50 mass%, and is more preferably 80 mass% to 100 mass%, in 100 mass% of the monomer (B3), from the viewpoint of excellent hydrophilicity of the polymer (C3).

(Polymerization of Monomer (B3) in Presence of Polymer (A))

The production method 3 is a method of polymerizing the monomer (B3) in the presence of the polymer (A).
It is preferable that the polymerization of the monomer (B3) in the presence of the polymer (A) is performed by a radical addition reaction from the viewpoint of being simple and of being industrially practically usable.
It is preferable that the polymer (A) includes a double bond from the viewpoint of having excellent reactivity.
The double bond of the polymer (A) can be introduced to the polymer (A) by using cross-linkable (meth)acrylate as a monomer in the polymerization for obtaining the polymer (A). In order to increase an introduction amount of the double bond of the polymer (A), a content rate of cross-linkable (meth)acrylate in the monomer that is used in the

polymerization for obtaining the polymer (A) may be increased.

In the polymerization of the monomer (B3) in the presence of the polymer (A), for example, the reaction is started by adding a radical generating agent.

Examples of the radical generating agent include a peroxide-based thermal radical generating agent such as tert-butyl hydroperoxide, cumene hydroperoxide, peroxyacetate, a peracetic acid, a chloroperbenzoic acid, ammonium persulfate, sodium persulfate, and potassium peroxodisulfate; an azo-based thermal radical generating agent such as a 4,4'-azobis(4-cynovaleric acid), 2,2'-azobis(2-methyl propione amidine) dihydrochloride, and a 2,2'-azobis[N-(2-carboxyethyl)-2-methyl propione amidine] n-hydrate; and a photoradical generating agent such as 1-[4-(2-hydroxyethoxy)-phenyl]-2-hydroxy-2-methyl propane. Only one type of such radical generating agents may be used, or two or more types thereof may be used together.

Among such radical generating agents, a radical generating agent in which the monomer (B3) is dissolved in a solvent described below is preferable, the azo-based thermal radical generating agent is more preferable, and 2,2'-azobis(2-methyl propione amidine) dihydrochloride and a 2,2'-azobis[N-(2-carboxyethyl)-2-methyl propione amidine] n-hydrate are even more preferable, from the viewpoint of having excellent reactivity.

In the polymerization of the monomer (B3) in the presence of the polymer (A), the solvent may be used, or the solvent may not be used, but it is preferable to use the solvent from the viewpoint of being capable of homogeneously dispersing the monomer (B3).

Examples of the solvent include water; ethers such as diethyl ether, tetrahydrofuran, and dioxane; hydrocarbons such as toluene and xylene; halogenated hydrocarbons such as halobenzene, dichloromethane, dichloroethane, and chloroform; alcohols such as methanol, ethanol, and isopropanol; nitriles such as acetonitrile; and polar solvents such as dimethyl formamide, dimethyl acetamide, dimethyl sulfoxide, and N-methyl-2-pyrrolidone. Only one type of such solvents may be used, or two or more types thereof may be used together. Among such solvents, the solvent in which the monomer (B3) is dissolved is preferable, water and a solvent that is mixed with water are more preferable, and water, acetonitrile, dimethyl formamide, and N-methyl-2-pyrrolidone are even more preferable, from the viewpoint of having excellent reactivity.

A polymerization temperature of the monomer (B3) in the presence of the polymer (A) is preferably 0°C to 100°C, and is more preferably 15°C to 90°C, from the viewpoint of having excellent reactivity.

A polymerization atmosphere of the monomer (B3) in the presence of the polymer (A) is not particularly limited, but may be an air atmosphere, or may be an inert gas atmosphere.

A polymerization time of the monomer (B3) in the presence of the polymer (A) is preferably 1 hour to 30 hours, and is more preferably 2 hours to 10 hours, from the viewpoint of sufficient progress of the reaction.

In the polymerization of the monomer (B3) in the presence of the polymer (A), it is preferable to use a chain transfer agent from the viewpoint of being capable of controlling a polymerization reaction.

Examples of the chain transfer agent include a mercaptan compound such as n-octyl mercaptan, n-dodecyl mercaptan, tert-dodecyl mercaptan, 1,4-butane dithiol, 1,6-hexane dithiol, ethylene glycol bisthiopropionate, butanediol bisthioglycolate, butanediol bisthiopropionate, hexanediol bisthioglycolate, hexanediol bisthiopropionate, trimethylol propane tris-(P-thiopropionate), pentaerythritol tetrakisthiopropionate, 2-mercaptoethanol, 3-mercapto-1,2-propanediol, aminoethanethiol, sodium 2-mercaptoethane sulfonate, and sodium 3-mercapto-1-propane sulfonate; an $\alpha$-methyl styrene dimer; terpinolene; and carbon tetrachloride. Only one type of such chain transfer agents may be used, or two or more types thereof may be used together.

Among such chain transfer agents, the mercaptan compound is preferable, and 2-mercaptoethanol, 3-mercapto-1,2-propanediol, aminoethanethiol, and sodium 3-mercapto-1-propane sulfonate are more preferable, from the viewpoint of easily controlling the polymerization reaction.

A used amount of the chain transfer agent is preferably 0.01 parts by mass to 5 parts by mass, and is more preferably 0.1 parts by mass to 3 parts by mass, with respect to 100 parts by mass of the monomer (B3), from the viewpoint of being capable of controlling the polymerization reaction.

A purification step such as solvent distillation, filtration, and washing may be provided after the polymerization of the monomer (B3) in the presence of the polymer (A).

As described above, the polymer (C3) (the hydrophilized (meth)acrylic polymer) is obtained by the polymerization of the monomer (B3) in the presence of the polymer (A).

[Polymer (C3)]

The polymer (C3) includes a structure represented by General Formula (4) described above.

In addition, the polymer (C3) includes a constitutional unit derived from the polymer (A) and the monomer (B3).

An addition amount of the constitutional unit derived from the monomer (B3) with respect to the polymer (A) in the polymer (C3) is preferably 1 part by mass to 30 parts by mass, and is more preferably 5 parts by mass to 25 parts by mass, with respect to 100 parts by mass of the polymer (A). In a case where the addition amount of the constitutional unit derived from the monomer (B3) with respect to the polymer (A) in the polymer (C3) is greater than or equal to 1 part by mass, the polymer (C3) has excellent hydrophilicity. In addition, in a case where the addition amount of the constitutional

unit derived from the monomer (B3) with respect to the polymer (A) in the polymer (C3) is less than or equal to 30 parts by mass, the purification step after the polymerization of the monomer (B3) in the presence of the polymer (A) can be easily performed.

A preferred shape of the polymer (C3) is the same as the preferred shape of the polymer (C1).

The polymer (C3) has excellent hydrophilicity, and thus, can be preferably used in a usage in which hydrophilicity is necessary, for example, can be preferably used in compound separating particles, a compound separating film, an antifouling resin plate, an antifogging resin plate, an antistatic plate, a resin modifier, and the like, and can be particularly preferably used for separating a compound.

The polymer (C3) may be further subjected to a treatment such as the modification of a functional group before being used as the usage.

In the case of using the polymer (C3) for separating a compound, porous particles and a porous film are preferable as the shape of the polymer (C3) from the viewpoint of having excellent separation capacity, and the porous particles are more preferable as the shape of the polymer (C3) from the viewpoint of being capable of easily separating a compound by filling a liquid chromatography column with the compound.

Preferred values of a modal fine pore radius of the porous particles, a fine pore volume, a specific surface area, a volume average particle diameter, and an average pore diameter of the porous film are the same as those in the case of the polymer (C1).

[Separating Agent]

The compound hydrophilized by the production method 3 can be used as a separating agent (hereinafter, may be referred to as a separating agent 3). The separating agent 3 is capable of including the structure represented by General Formula (2) described above.

In addition, the structure represented by General Formula (2) is preferably the structure represented by General Formula (4) described above, is more preferably the structure represented by General Formula (4a) described above, and is even more preferably the structure represented by General Formula (4b).

In addition, the separating agent 3, for example, can be obtained by the production method 3 described above.

A preferred shape of the separating agent 3 is the same as the preferred shape of the polymer (C1).

[Separation and Production of Compound]

As described above, the polymers obtained in the first embodiment to the third embodiment of the invention can be preferably used for separating a compound, and a compound after separation can be obtained by the separation.

Among the compounds, protein and peptide are preferable from the viewpoint of being more preferably usable in separation.

In the polymers obtained in the first embodiment to the third embodiment of the invention, only one polymer may be used, or a plurality of polymers may be simultaneously used.

That is, the polymer obtained in the first embodiment of the invention can be used together with at least one of the polymer obtained in the second embodiment of the invention and the polymer obtained in the third embodiment of the invention.

The polymer obtained in the second embodiment of the invention can be used together with at least one of the polymer obtained in the first embodiment of the invention and the polymer obtained in the third embodiment of the invention.

The polymer obtained in the third embodiment of the invention can be used together with at least one of the polymer obtained in the first embodiment of the invention and the polymer obtained in the second embodiment of the invention.

In each of the production methods according to the first embodiment to the third embodiment of the invention, only one production method may be performed, or a plurality of production methods may be sequentially performed. In a case where a plurality of production methods are performed, the order is not particularly limited.

That is, the production method according to the second embodiment or the production method according to the third embodiment may be further performed before and after the production method according to the first embodiment of the invention.

The production method according to the first embodiment or the production method according to the third embodiment may be further performed before and after the production method according to the second embodiment of the invention.

The production method according to the first embodiment or the production method according to the second embodiment may be further performed before and after the production method according to the third embodiment of the invention.

## Examples

The invention will be described in detail by using the following examples, but the invention is not limited thereto.

Note that, symbols in polymer structures in Tables 1 to 3 respectively correspond to symbols in General Formula (3) or (4).

[Test Example 1]

(Comparative Example 1-1)

0.008 parts by mass of sodium dodecyl sulfate and 498 parts by mass of water were added to 1.25 parts by mass of polymethyl methacrylate seed particles (an average particle diameter of 2.0 μm), and thus, an aqueous

dispersion of seed particles was prepared. 4.55 parts by mass of sodium dodecyl sulfate and 1150 parts by mass of water were added to 100 parts by mass of ethylene glycol dimethacrylate, 150 parts by mass of toluene, and 2 parts by mass of 2,2'-azobisisobutyronitrile, and thus, an aqueous dispersion of a monomer was prepared. The prepared aqueous dispersion of the monomer was added to the prepared aqueous dispersion of the seed particles, and stirring was performed at 25°C for 24 hours, and thus, the monomer or the like was absorbed in the seed particles.

Next, 430 parts by mass of an aqueous solution of 5 mass% of polyvinyl alcohol (Product Name "GOHSENOL GH-20", manufactured by Mitsubishi Chemical Corporation), 0.215 parts by mass of sodium nitrite, and 71 parts by mass of water were added to the dispersion, and polymerization was performed at 70°C for 3 hours. The obtained polymer was isolated, and a polymer derived from the seed particles was removed by toluene extraction, and drying was performed, and thus, a polymer of porous particles was obtained.

The obtained polymer was subjected to the following measurement. A modal fine pore radius was 197 angstroms, a fine pore volume was 0.97 mL/g, a specific surface area was 450 $m^2/g$, and a volume average particle diameter was 10 μm.

The obtained polymer was subjected to the following hydrophilic evaluation result. In addition, a recovery rate of bovine serum albumin (BSA) was measured by the following method. Results are shown in Table 1.

(Example 1-1)

100 parts by mass of the polymer obtained in Comparative Example 1-1, 1900 parts by mass of water, 103 parts by mass of 3-mercapto-1,2-propanediol, and 39 parts by mass of a 2,2'-azobis[N-(2-carboxyethyl)-2-methyl propione diamine] tetrahydrate (Product Name "VA-057", manufactured by FUJIFILM Wako Pure Chemical Corporation) were mixed, and a reaction was performed at 70°C for 5 hours in a nitrogen atmosphere. A reaction liquid was cooled, and then, the obtained polymer was washed with water, and filtration was performed, and thus, a polymer was obtained.

The obtained polymer was subjected to the following hydrophilic evaluation result. In addition, a recovery rate of bovine serum albumin (BSA) was measured by the following method. Results are shown in Table 1.

(Example 1-2)

100 parts by mass of the polymer obtained in Example 1-1, 490 parts by mass of water, and 276 parts by mass of a sodium hypochlorite solution (manufactured by FUJIFILM Wako Pure Chemical Corporation) were mixed, and pH of the solution was adjusted to 10 to 11, and then, a reaction was performed at 30°C for 5 hours. A reaction liquid was cooled, and then, the obtained polymer was washed with water, and filtration was performed, and thus, a polymer was obtained.

The obtained polymer was subjected to the following hydrophilic evaluation result. Results are shown in Table 1.

(Example 1-3)

100 parts by mass of the polymer obtained in Comparative Example 1-1, 1900 parts by mass of water, 74 parts by mass of aminoethanethiol, and 3.16 parts by mass of a 2,2'-azobis[N-(2-carboxyethyl)-2-methyl propione diamine] tetrahydrate (Product Name "VA-057", manufactured by FUJIFILM Wako Pure Chemical Corporation) were mixed, and a reaction was performed at 70°C for 5 hours in a nitrogen atmosphere. A reaction liquid was cooled, and then, the obtained polymer was washed with water, and filtration was performed, and thus, a polymer was obtained.

The obtained polymer was subjected to the following hydrophilic evaluation result. Results are shown in Table 1.

(Example 1-4)

100 parts by mass of the polymer obtained in Comparative Example 1-1, 1667 parts by mass of water, 149 parts by mass of sodium 3-mercapto-1-propane sulfonate, and 35 parts by mass of a 2,2'-azobis[N-(2-carboxyethyl)-2-methyl propione diamine] tetrahydrate (Product Name "VA-057", manufactured by FUJIFILM Wako Pure Chemical Corporation) were mixed, and a reaction was performed at 70°C for 5 hours in a nitrogen atmosphere. A reaction liquid

was cooled, and then, the obtained polymer was washed with water, and filtration was performed, and thus, a polymer was obtained.

The obtained polymer was subjected to the following hydrophilic evaluation result. Results are shown in Table 1.

[Each Evaluation Method and Measurement Method] (Hydrophilic Evaluation 1)

A column (Product Name "Empty Column (Stainless Steel)", manufactured by Sugiyama Shoji Co., Ltd, an inner diameter of 4.6 mm and a height of 150 mm) was filled with the polymers obtained in the examples and the comparative examples, and a retention time of an authentic sample was measured in the following hydrophilizing evaluation condition.

Hydrophilic Evaluation Condition

Evaluation Device: an HPLC system (manufactured by Shimadzu Corporation)
Authentic Sample: dimethyl phthalate, diethyl phthalate, and dipropyl phthalate
Eluent A: Acetonitrile/Water = 70/30 (volume ratio)
Eluent B: water
Elution Condition: a mixture of 55 volume% of an eluent A and 45 volume% of an eluent B
Flow Rate: 1 mL/minute
Column Temperature: 60°C
Measurement Wavelength: 254 nm
Sample Injection Amount: 5 μL

(Hydrophilic Evaluation 2)

Approximately 5 g of water was added to approximately 1 mg of the polymer obtained in the examples and the comparative examples, stirring was performed, and a zeta potential was measured by using a zeta potential measurement device (Model Name "Zetasizer Nano ZS", manufactured by Malvern Panalytical Ltd).

(Recovery Rate of BSA)

A column (Product Name "Empty Column (Stainless Steel)", manufactured by Sugiyama Shoji Co., Ltd, an inner diameter of 4.6 mm and a height of 150 mm) was filled with the polymer obtained in the examples and the comparative examples, and a recovery rate of BSA was measured in the following condition.

Preparative Condition

Preparative Device: AKTA avant 25 (manufactured by GE Healthcare Inc.)
Sample S: BSA was adjusted with an eluent A such that a concentration was 1 g/L
Eluent A: 20 mM Tris-HCl (pH 8.0)
Eluent B: 20 mM Tris-HCl + 1 M NaCl (pH 8.0)
Elution Condition: S (49.8 mL) → A (24.9 mL) → B (24.9 mL)
Flow Rate: 0.83 mL/minute
Column Temperature: 20°C
Evaluation Condition

A light absorbance of each preparative solution was measured, the amount of contained BSA was calculated, and a recovery rate of BSA was calculated by the following expression.

$$\text{Recovery Rate of BSA} = \text{Amount (mg) of BSA Contained in Preparative Sample S, Eluent A, and Eluent B} / \text{Amount (mg) of injected BSA} \times 100(\%)$$

Evaluation Device: an ultraviolet-visible spectrophotometer UV-1850 (manufactured by Shimadzu Corporation)
Measurement Wavelength: 280 nm
Cell: a cell of 1 cm

(Measurement of Modal Fine Pore Radius)

The polymer obtained in Comparative Example 1-1 was weighed, adsorption-desorption isotherm was measured by using a specific surface area-fine pore distribution measurement device (Model Name "ASAP 2420 Type", manufactured by Micromeritics Instrument Corporation), a cumulative fine pore volume distribution and a Log differential fine pore volume distribution were plotted from the obtained adsorption-desorption isotherm, and a modal fine pore radius was calculated.
Note that, the modal fine pore radius of the polymer obtained in Comparative Example 1-1 and the modal fine pore radius of the polymer obtained in each example are regarded as the same modal fine pore radius.

(Measurement of Fine Pore Volume)

The polymer obtained in Comparative Example 1-1 was weighed, adsorption-desorption isotherm was measured by using a specific surface area-fine pore distribution measurement device (Model Name "ASAP 2420 Type", manufactured by Micromeritics Instrument Corporation), a cumulative fine pore volume distribution and a Log differential fine pore volume distribution were plotted from the obtained adsorption-desorption isotherm, and a fine pore volume was calculated.
Note that, the fine pore volume of the polymer obtained in Comparative Example 1-1 and the fine pore volume of the polymer obtained in each example are regarded as the same fine pore volume.

(Measurement of Specific Surface Area)

The polymer obtained in Comparative Example 1-1 was weighed, and a specific surface area was calculated by using a specific surface area-fine pore distribution measurement device (Model Name "ASAP 2420 Type", manufactured by Micromeritics Instrument Corporation).
Note that, the specific surface area of the polymer obtained in Comparative Example 1-1 and the specific surface area of the polymer obtained in each example are regarded as the same specific surface area.

(Measurement of Volume Average Particle Diameter)

A volume average particle diameter of the polymer obtained in Comparative Example 1-1 was obtained by measuring a particle diameter of arbitrary 400 polymers with an optical microscope (Model Name "ECLIPSE LV100ND", manufactured by NIKON CORPORATION), and by calculating a volume median size from the distribution.
Note that, the volume average particle diameter of the polymer obtained in Comparative Example 1-1 and the volume average particle diameter of the polymer obtained in each example are regarded as the same volume average particle diameter.
[Table 1]

Table 1

| | Thiol compound (B1) | Polymer structure | | | | Retention time [min] | | | Zetapotential [mV] | Recovery rate [%] of BSA |
|---|---|---|---|---|---|---|---|---|---|---|
| | | $X_{31}$ | $Y_{31}$ | $Y_{32}$ | n | Dimethyl phthalate | Diethyl phthalate | Dipropyl phthalate | | |
| Example 1-1 | 3-Mercapto-1,2-propanediol | $CHOHCH_2OH$ | H | H | 0 | 3.417 | 4.193 | 6.069 | -32.5 | 55.5 |
| Example 1-2 | 3-Mercapto-1,2-propanediol | $CHOHCH_2OH$ | H | H | 0 to 2 | 3.374 | 4.097 | 5.843 | - | - |
| Example 1-3 | Aminoethanethiol | $-CH_2NH_2$ | H | H | 0 | 3.237 | 3.870 | 5.380 | - | - |
| Example 1-4 | Sodium 3-mercapto-1-propane sulfonate | $-C_2H_4SO_3Na$ | H | H | 0 | 3.087 | 3.660 | 5.196 | - | - |
| Comparative Example 1-1 | - | - | - | - | - | 5.129 | 7.696 | 14.116 | -29.8 | 2.9 |

[Test Example 2]

(Comparative Example 2-1)

A polymer of porous particles was obtained by the same method as that in Comparative Example 1-1.

(Example 2-1)

100 parts by mass of the polymer obtained in Comparative Example 2-1, 1312 parts by mass of tetrahydrofuran, and 308 parts by mass of ethanol amine were mixed, and a reaction was performed at 68°C for 5 hours in a nitrogen atmosphere. A reaction liquid was cooled, and then, the obtained polymer was washed with water, and filtration was performed, and thus, a polymer was obtained.

The obtained polymer was subjected to the hydrophilic evaluation result described in Test Example 1. In addition, a recovery rate of bovine serum albumin (BSA) was measured by the method described in Test Example 1. Results are shown in Table 2.

(Example 2-2)

100 parts by mass of the polymer obtained in Comparative Example 2-1, 1090 parts by mass of ethanol, and 530 parts by mass of diethanol amine were mixed, and a reaction was performed at 85°C for 5 hours in a nitrogen atmosphere. A reaction liquid was cooled, and then, the obtained polymer was washed with water, and filtration was performed, and thus, a polymer was obtained.

The obtained polymer was subjected to the hydrophilic evaluation result described in Test Example 1. Results are shown in Table 2.

[Table 2]

Table 2

| | Aminoalcoholcompound (B2) | Polymer structure | | | | Retention time [min] | | | Recovery rate [%] of BSA |
|---|---|---|---|---|---|---|---|---|---|
| | | $X_{41}$ | $Y_{41}$ | $Y_{42}$ | $Y_{43}$ | Dimethyl phthalate | Diethyl phthalate | Dipropyl phthalate | |
| Example 2-1 | Ethanol amine | $-CH_2OH$ | H | H | H | 4.197 | 5.711 | 9.458 | 17.8 |
| Example 2-2 | Diethanol amine | $-CH_2OH$ | H | H | $-C_2H_4OH$ | 4.398 | 6.194 | 10.632 | - |
| Comparative Example 2-1 | - | - | - | - | - | 5.129 | 7.696 | 14.116 | 2.9 |

[Test Example 3]

(Comparative Example 3-1)

A polymer of porous particles was obtained by the same method as that in Comparative Example 1-1.

(Example 3-1)

100 parts by mass of the polymer obtained in Comparative Example 3-1, 892 parts by mass of water, 57 parts by mass of hydroxyethyl acrylamide, 0.54 parts by mass of 3-mercapto-1,2-propanediol, and 1.72 parts by mass of a 2,2'-azobis(2-carboxyethyl)2-methyl propione amidine tetrahydrate (Product Name "VA-057", manufactured by FUJIFILM Wako Pure Chemical Corporation) were mixed, and a reaction was performed at 80°C for 5 hours in a nitrogen atmosphere. A reaction liquid was cooled, and then, the obtained polymer was washed with desalinated water, and filtration was performed, and thus, a polymer was obtained.

The obtained polymer was subjected to the hydrophilic evaluation result described in Test Example 1. In addition, a recovery rate of bovine serum albumin (BSA) was measured by the method described in Test Example 1. Results are shown in Table 3.

(Example 3-2)

100 parts by mass of the polymer obtained in Comparative Example 3-1, 860 parts by mass of water, 138 parts by mass of a dimethyl aminopropyl acrylamide methyl chloride quaternary salt, 0.54 parts by mass of 3-mercapto-1,2-propanediol, and 1.72 parts by mass of a 2,2'-azobis(2-carboxyethyl)2-methyl propione amidine tetrahydrate (Product Name "VA-057", manufactured by FUJIFILM Wako Pure Chemical Corporation) were mixed, and a reaction was performed at 80°C for 5 hours in a nitrogen atmosphere. A reaction liquid was cooled, and then, the obtained polymer was washed with desalinated water, and filtration was performed, and thus, a polymer was obtained.

The obtained polymer was subjected to the hydrophilic evaluation result described in Test Example 1. Results are shown in Table 3.

(Example 3-3)

100 parts by mass of the polymer obtained in Comparative Example 3-1, 1000 parts by mass of water, 103 parts by mass of sodium p-styrene sulfonate, 3.56 parts by mass of sodium 3-mercapto-1-propane sulfonate, and 2.07 parts by mass of a 2,2'-azobis(2-carboxyethyl)2-methyl propione amidine tetrahydrate (Product Name "VA-057", manufactured by FUJIFILM Wako Pure Chemical Corporation) were mixed, and a reaction was performed at 80°C for 5 hours in a nitrogen atmosphere. A reaction liquid was cooled, and then, the obtained polymer was washed with desalinated water, and filtration was performed, and thus, a polymer was obtained.

The obtained polymer was subjected to the hydrophilic evaluation result described in Test Example 1. Results are shown in Table 3.

[Table 3]

Table 3

| | Monomer (B3) | Polymer structure | | | | Retention time [min] | | | Zeta potential [mV] | Recovery rate [%] of BSA |
|---|---|---|---|---|---|---|---|---|---|---|
| | | $X_{41}$ | $Y_{41}$ | $Y_{42}$ | $Y_{43}$ | Dimethyl phthalate | Diethyl phthalate | Dipropyl phthalate | | |
| Example 3-1 | Hydroxyethyl acrylamide | $-CH_2OH$ | H | H | H | 3.397 | 4.365 | 6.891 | -32.6 | 62.7 |
| Example 3-2 | Dimethyl aminopropyl acrylamide methyl chloride quaternary salt | $(CH_2)_2N^+(CH_3)_3$ | H | H | H | 3.264 | 4.344 | 7.322 | - | - |
| Example 3-3 | Sodium p-styrene sulfonate | - | - | - | - | 2.997 | 3.777 | 5.987 | - | - |
| Comparative Example 3-1 | - | - | - | - | - | 5.129 | 7.696 | 14.116 | -29.8 | 2.9 |

The invention has been described in detail with reference to specific embodiments, but it is obvious to a person skilled in the art that various modifications or corrections which do not depart from the gist and the scope of the invention can be added. The present application is based on a Japanese patent application filed on March 20, 2019 (Japanese Patent Application No. 2019-052188), a Japanese patent application filed on March 20, 2019 (Japanese Patent Application No. 2019-052189), a Japanese patent application filed on August 29, 2019 (Japanese Patent Application No. 2019-156263), and a Japanese patent application filed on August 29, 2019 (Japanese Patent Application No. 2019-156264), and the contents of which are incorporated herein by reference.

## Claims

1. A polymer including at least one structure selected from the group consisting of a structure represented by General Formula (3) described below and a structure represented by General Formula (4) described below:

[Chemical Formula 1]

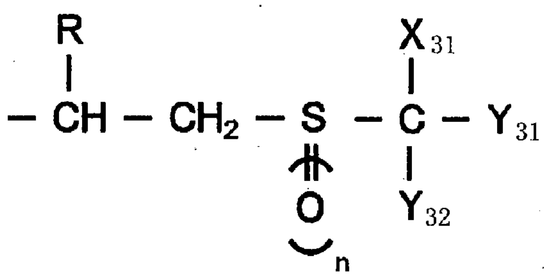

(3)

in General Formula (3) described above, $X_{31}$ represents a hydrophilic group-containing structure, $Y_{31}$ and $Y_{32}$ each independently represent a hydrophilic group-containing structure, a hydrogen atom, or an alkyl group, n represents an integer of 0 to 2, and R represents a hydrogen atom or an alkyl group; and

[Chemical Formula 2]

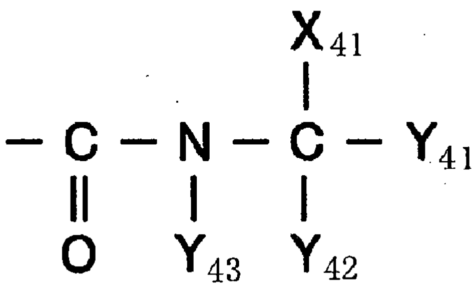

(4)

in General Formula (4) described above, $X_{41}$ represents a hydrophilic group-containing structure, and $Y_{41}$ to $Y_{43}$ each independently represent a hydrophilic group-containing structure, a hydrogen atom, or an alkyl group.

2. The polymer according to claim 1, wherein the hydrophilic group includes at least one selected from the group consisting of a hydroxyl group, a carboxyl group, a sulfo group, and an amino group.

3. A separating agent including at least one structure selected from the group consisting of a structure represented by General Formula (1) described below and a structure represented by General Formula (2) described below:

[Chemical Formula 3]

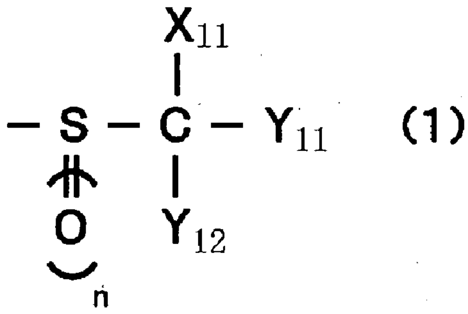

in General Formula (1) described above, $X_{11}$ represents a hydrophilic group-containing structure, $Y_{11}$ and $Y_{12}$ each independently represent a hydrophilic group-containing structure, a hydrogen atom, or an alkyl group, and n represents an integer of 0 to 2; and

[Chemical Formula 4]

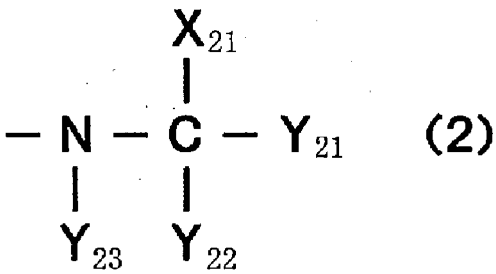

in General Formula (2) described above, $X_{21}$ represents a hydrophilic group-containing structure, and $Y_{21}$ to $Y_{23}$ each independently represent a hydrophilic group-containing structure, a hydrogen atom, or an alkyl group.

**4.** The separating agent according to claim 3,
wherein the hydrophilic group includes at least one selected from the group consisting of a hydroxyl group, a carboxyl group, a sulfo group, and an amino group.

**5.** A production method of a polymer, including at least one method selected from the group consisting of methods (1) to (3) described below:

a method (1) of obtaining a polymer (C1) by reacting a (meth)acrylic polymer (A) with a thiol compound (B1) having a hydrophilic group in accordance with a thiol-ene reaction;
a method (2) of obtaining a polymer (C2) by reacting the (meth)acrylic polymer (A) with an aminoalcohol compound (B2) in accordance with an ester exchange reaction; and
a method (3) of obtaining a polymer (C3) by polymerizing a monomer (B3) including at least one selected from the group consisting of a sulfonic acid having a vinyl group and N-substituted (meth)acrylamide in the presence of the (meth)acrylic polymer (A).

**6.** The production method of a polymer according to claim 5,
wherein the polymer (A) includes a cross-linked structure.

**7.** The production method of a polymer according to claim 5 or 6,
wherein in the method (1), the polymer (C1) is obtained through an oxidation step after the reaction between the polymer (A) and the compound (B1).

**8.** The production method of a polymer according to any one of claims 5 to 7,

wherein in the method (1), the hydrophilic group includes at least one selected from the group consisting of a hydroxyl group, a carboxyl group, a sulfo group, and an amino group.

**9.** The production method of a polymer according to any one of claims 5 to 8, wherein in the method (1), the compound (B1) includes at least one selected from the group consisting of 2-mercaptoethanol, 3-mercapto-1,2-propanediol, aminoethanethiol, and sodium 3-mercapto-1-propane sulfonate.

**10.** The production method of a polymer according to claim 5 or 6, wherein in the method (2), the compound (B2) includes at least one selected from the group consisting of ethanol amine, propanol amine, N-(3-aminopropyl) diethanol amine, 3-amino-1,2-propanediol, and diethanol amine.

**11.** The production method of a polymer according to claim 5 or 6, wherein in the method (3), the monomer (B3) includes at least one selected from the group consisting of a hydroxyl group, a carboxyl group, a sulfo group, and an amino group.

**12.** The production method of a polymer according to claim 5, 6, or 11, wherein in the method (3), the N-substituted (meth)acrylamide includes at least one selected from the group consisting of hydroxyethyl (meth)acrylamide, hydroxypropyl (meth)acrylamide, and a dimethyl aminopropyl (meth)acrylamide methyl chloride quaternary salt.

**13.** The production method of a polymer according to claim 5, 6, 11, or 12, wherein in the method (3), the sulfonic acid having a vinyl group includes sodium p-styrene sulfonate.

**14.** A separation method of a compound, using the polymer according to claim 3 or 4 for separating a compound.

**15.** A separation method of a compound, using a polymer obtained by the production method according to any one of claims 5 to 13 for separating a compound.

**16.** A production method of a compound, including the separation method according to claim 14 or 15.

## INTERNATIONAL SEARCH REPORT

International application No.
PCT/JP2020/011306

A. CLASSIFICATION OF SUBJECT MATTER
Int.Cl. B01D71/40(2006.01)i, C08F8/32(2006.01)i, C08F8/34(2006.01)i, C08F265/06(2006.01)i, C08F2/44(2006.01)i, B01D15/38(2006.01)i, C08G75/045(2016.01)i, B01J20/26(2006.01)i, B01J20/281(2006.01)i, B01J20/30(2006.01)i
FI: C08F8/34, C08F8/32, C08G75/045, C08F2/44C, B01J20/26L, B01J20/30, B01D71/40, B01D15/38, B01J20/26H, C08F265/06
According to International Patent Classification (IPC) or to both national classification and IPC

B. FIELDS SEARCHED
Minimum documentation searched (classification system followed by classification symbols)
Int.Cl. B01D71/40, C08F8/32, C08F8/34, C08F265/06, C08F2/44, B01D15/38, C08G75/045, B01J20/26, B01J20/281, B01J20/30

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2018/147393 A1 (MITSUBISHI CHEMICAL HOLDINGS CORP.) 16.08.2018 (2018-08-16), claims, examples 1-1, 1-3, 1-8, 1-11 | 1-6, 8, 9, 11, 12, 14-16 |
| X | JP 2018-155745 A (MITSUBISHI CHEMICAL HOLDINGS CORP.) 04.10.2018 (2018-10-04), claims, examples, paragraphs [0081]-[0090] | 1-6, 11-16 |
| X | JP 2018-155730 A (MITSUBISHI CHEMICAL HOLDINGS CORP.) 04.10.2018 (2018-10-04), claims, examples, paragraphs [0080]-[0082] | 1-6, 8, 9, 11-16 |
| X | WO 2015/119255 A1 (JSR CORPORATION) 13.08.2015 (2015-08-13), claims, examples, paragraph [0260] | 1-9, 14-16 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>19.05.2020 | Date of mailing of the international search report<br>26.05.2020 |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

International application No. PCT/JP2020/011306

C (Continuation). DOCUMENTS CONSIDERED TO BE RELEVANT

| Category* | Citation of document, with indication, where appropriate, of the relevant passages | Relevant to claim No. |
|---|---|---|
| X | WO 2015/080174 A1 (JSR CORPORATION) 04.06.2015 (2015-06-04), claims, examples | 1-9, 14-16 |
| X | JP 7-88366 A (MITSUBISHI CHEMICAL CORPORATION) 04.04.1995 (1995-04-04), claims, examples | 1-6, 11-16 |
| X | JP 55-142504 A (SUMITOMO CHEM CO., LTD.) 07.11.1980 (1980-11-07), claims, example 1 | 1-6, 10, 14-16 |
| X | JP 2015-54891 A (DIC CORP.) 23.03.2015 (2015-03-23), claims 1, 3, examples | 1, 2, 5, 6, 10 |
| X | JP 57-80403 A (TOAGOSEI CHEM IND CO., LTD.) 20.05.1982 (1982-05-20), claims, examples | 1, 2, 5, 6, 10 |

Form PCT/ISA/210 (second sheet) (January 2015)

**INTERNATIONAL SEARCH REPORT**
Information on patent family members

International application No.
PCT/JP2020/011306

| | | |
|---|---|---|
| WO 2018/147393 A1 | 16.08.2018 | US 2019/0359649 A1<br>claims, examples 1-1, 1-3, 1-8, 1-11<br>EP 3581578 A2 |
| JP 2018-155745 A | 04.10.2018 | (Family: none) |
| JP 2018-155730 A | 04.10.2018 | (Family: none) |
| WO 2015/119255 A1 | 13.08.2015 | US 2017/0043320 A1<br>claims, examples, paragraph [0213]<br>EP 3076170 A2<br>KR 10-2016-0078451 A<br>CN 105992949 A |
| WO 2015/080174 A1 | 04.06.2015 | US 2017/0043320 A1<br>claims, examples<br>EP 3076170 A2<br>KR 10-2016-0078451 A<br>CN 105992949 A |
| JP 7-88366 A | 04.04.1995 | (Family: none) |
| JP 55-142504 A | 07.11.1980 | (Family: none) |
| JP 2015-54891 A | 23.03.2015 | (Family: none) |
| JP 57-80403 A | 20.05.1982 | US 4269760 A<br>claims, examples<br>US 4314932 A<br>GB 2029425 A<br>DE 2927249 A<br>FR 2430427 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S6055009 A
- JP S54160300 A
- JP 2014210888 A
- JP 2019052188 A
- JP 2019052189 A
- JP 2019156263 A
- JP 2019156264 A